# EUROPEAN PATENT APPLICATION

(11) **EP 4 425 427 A1**
(43) Date of publication of application: **04.09.2024**
(21) Application number: 23159152.0
(22) Date of filing: 28.02.2023
(51) Int. Cl.: G06T 7/11, G06T 3/40

(54) **METHOD FOR AUTOMATED PROCESSING OF VOLUMETRIC MEDICAL IMAGES**

(71) Applicant: Siemens Healthineers AG, 91301 Forchheim (DE)
(72) Inventor: YEREBAKAN, Halid, Carmel, IN 46033 (US); HERMOSILLO VALADEZ, Gerardo, West Chester, PA 19382 (US); SHINAGAWA, Yoshihisa, Downingtown, PA 19335 (US)
(74) Representative: Horn Kleimann Waitzhofer Schmid-Dreyer Patent- und Rechtsanwälte PartG mbB

(57) **Abstract**

A method, device and system for automated processing of volumetric medical images (MI). In one aspect, the method comprises:
receiving (301) a volumetric medical image (MI), the volumetric medical image (MI) comprising at least one organ or portion thereof,
providing (302) a sparse sampling model (SM) for sparse sampling the volumetric medical image (MI), the sparse sampling model (SM) defining a number N of sampling points (400a-400c) distributed in the volumetric medical image (MI) and defining locations and distances of the distributed sampling points (400a-400c),
sampling (303) voxels (306-308) from the volumetric medical image (MI) using the provided sparse sampling model (SM) for obtaining N sparse sampling descriptors (D),
classifying (304) labels (L) for query points (701) in the volumetric medical image (MI) by applying a trained classifier to the obtained sparse sampling descriptors (D), and
providing (305) a segmentation mask (MAP, 601, 602) for the volumetric medical image (MI) using the classified labels (L).

## Description

The present invention relates to a computer-implemented method for automated processing of volumetric medical images, to a device, to a system, to a computer program product and to a computer-readable medium.

Segmentation is one of the core problems in medical imaging. It has been used for identifying organ boundaries, displaying visualizations or volume calculations. One prominent use case is organ identification in the finding location. However, full segmentation of all organs for this purpose is computationally intensive since 3D images could contain up to billions of voxels. Also, the identification of organs in a location of interest does not need full organ segmentation in most cases.

As compared to landmarking and bounding box detection methods, segmentation provides more granular information, which would be beneficial while storing anatomical locations of findings in structured databases, quantification of abnormalities, radio therapy planning, dose calculations, comparing longitudinal studies, visualization of imaging data or filter the scanning region for CAD algorithms. Thus, it is desired to have fast and accurate segmentation algorithms.

Conventional methods are known from references [1] to [8], as shown below. From these recent methods, U-net is a widely used machine learning algorithm that learns the mapping from image to segmentation space. The power of U-net comes from utilizing multiple resolutions of the same image while extracting feature vectors. Thus, coarse layers work on global representation, while finer levels work on local details. Further, conventional transformer-based encoder-decoder approaches have been demonstrated to be more powerful in biomedical segmentation tasks. These methods usually depend on large training datasets with a considerable amount of training time with specialized hardware. Monai Label is a software that provides intelligent segmentation tools. It enables labeling and learning at the same time. The segmentation results could be visualized in 3D as well.

Flare challenge created an open competition for abdominal organ segmentation under low resources. Higher performing methods in that challenge were demonstrated to be around 10s in time scale in the segmentation tasks.

The object of the invention is therefore to provide a method, device and system that enables an improved automated processing of volumetric medical images.

According to a first aspect, a computer-implemented method for automated processing of volumetric medical images is provided. The method comprises:
a) receiving a volumetric medical image, the volumetric medical image comprising at least one organ or portion thereof,
b) providing a sparse sampling model for sparse sampling the volumetric medical image, the sparse sampling model defining a number N of sampling points distributed in the volumetric medical image and defining locations and distances of the distributed sampling points, wherein "N" is a positive, real and whole number,
c) sampling voxels from the volumetric medical image using the provided sparse sampling model for obtaining N sparse sampling descriptors,
d) classifying labels for query points in the volumetric medical image by applying a trained classifier to the obtained sparse sampling descriptors, and
e) providing a segmentation mask for the volumetric medical image using the classified labels.

As a result, the segmentation mask is provided using sparse sampling and sparse sampling descriptors. Even though the amount of data processed is reduced, the inventors found that the segmentation mask can still be provided in good quality, and a type of organ can particularly be identified reliably using the present approach.

As the data processed is reduced, the present approach is very fast in providing a segmentation mask. As the user may define and/or adapt the query points for classifying the labels, the user has the ability to define the granularity of the segmentation mask. For example, a coarse segmentation mask may be provided very fast. Furthermore, a fine segmentation mask may be provided with detailed information for the user.

For example, by using query points in every 8 mm, a coarse segmentation mask may be obtained in around one second. In the segmentation mask, each visualized intensity may represent a different organ label.

The provided segmentation mask may be used for identifying organ boundaries, displaying visualizations and/or volume calculations. The term "segmentation mask" may be also referred to as "segmentation map".

An organ is to be understood as a collection of tissue joined in a structural unit to serve a common function. The organ may be a human organ. The organ may be any one of the following, for example: intestines, skeleton, kidneys, gall bladder, liver, muscles, arteries, heart, larynx, pharynx, brain, lymph nodes, lungs, spleen bone marrow, stomach, veins, pancreas, and bladder.

The volumetric medical image may be captured by and received from a medical imaging unit, the medical imaging unit may include, for example, but not limited to, a magnetic resonance imaging device, a computer tomography device, an X-ray imaging device, an ultrasound imaging device, etc. The volumetric medical image may be three-dimensional (3D) and/or related to a volume. The volumetric medical image may be made up of a number of slices, i.e., 2D (two-dimensional) medical images. The 2D medical images may be captured by and received from the medical imaging unit mentioned above. The 2D medical images may then be assembled to form the volumetric medical image.

Presently, a voxel represents a value in three-dimensional space, whereas a pixel represents a value in two-dimensional space. The pixels or voxels may or may not have their position, i.e., their coordinates explicitly encoded with their values. Instead, the position of a pixel or voxel is inferred based upon its position relative to other pixels or voxels (i.e., is positioned in the data structure that makes up a single 2D or 3D (volumetric) image). The voxels may be arranged on a 3D grid, the pixels on a 2D grid. The 2D medical image may, for example, be in the form of an array of pixels. The volumetric medical image may comprise an array of voxels. The pixels of a number of 2D medical images making up a volumetric medical image are also presently referred to as voxels. The pixels or voxels may be representative of intensity, absorption or other parameters as a function of a three-dimensional position, and may, for example, be obtained by a suitable processing of measurement signals obtained by one or more of the above-mentioned medical imaging units.

"Sparse sampling" is to be understood as, when having regard to the total number of voxels making up the volumetric medical image, only few voxels being used in sampling. In particular, "sparse" is to say that less than 50% or less than 20% or even less than 10% of the total number of voxels of the volumetric medical image are sampled in step c). Sparse sampling has the effect that the amount of data which needs to be processed by the trained classifier is reduced, thus reducing computation time and computation resources. Even though the amount of data processed by the trained classifier is reduced, the inventors found that the segmentation mask can still be provided in good quality, and a type of organ can particularly be identified reliably using the present approach. One reason for this is that the sampled voxels may correspond to a larger field of view, thus also considering neighborhood information, compared to the case where every voxel of a smaller sub volume is sampled. The sampling model contains the information about the location of the voxels in the volumetric medical image which are to be sampled, i.e. the sampling points distributed in the volumetric medical image and the locations and distances of the distributed sampling points The sampling model can be or make use of an algorithm, for example.

The respective sparse sampling descriptor may be formed as a vector of values, in particular of intensities, of the sampled voxels associated to a certain sampling point of the distributed sampling points.

A trained classifier is applied to the sparse sampling descriptors obtained in step c). The trained classifier is, for example, a trained neural network.

In one embodiment, the method further comprises:
identifying the type of organ at a certain point of interest, in particular by applying the trained classifier to the sampled voxels.

In particular, a robot, (e.g., CT or MR) scanner or other device or machine is controlled depending on the identified type of organ (or organ specific abnormality as discussed below). The robot may be configured for operating on a patient's body, for example. In particular, a robot (e.g., an operating instrument thereof such as a scalpel) or scanner movement may be controlled depending on the identified organ.

In a further embodiment, the method further comprises:
receiving a command, in particular a user input, for determining the certain point of interest, wherein the sparse sampling model is provided in dependence on the received command.

Thus, the point of interest may be selected by a user. For example, the point of interest can be selected using a graphical user interface and an input device, such as a pointer device, to interact with the graphical user interface to select the point of interest. In another embodiment, the point of interest may be input using a keyboard, a data file or the like. According to a further embodiment, the point of interest is selected by pausing a cursor operated by the user on the volumetric medical image or a part thereof displayed on the graphical user interface. "Pausing" here means that the cursor is not moved by the operator. This allows for a quick and efficient analysis of a volumetric medical image by a user, for example a doctor.

In a further embodiment, the sparse sampling model is provided such that the voxels are sampled with a sampling rate per unit length, area or volume which decreases with a distance of the respective voxel on the certain point of interest.

In particular, the sampling rate decreases at a non-linear rate, in particular at the rate of an exponential logarithmic or a power function. The inventors found that using a sampling rate as described reduces computation time significantly, while, at the same time, providing the segmentation mask reliably.

In particular, the sampled voxels are less than 1 %, preferably less than 0,1 %, and more preferably less than 0,01 % of the total number of voxels in the volumetric medical image.

In a further embodiment, the sparse sampling model defines a plurality of grids, in particular 3D grids, of different grid spacings, the different grid spacings determining different distances of the distributed sampling points in the volumetric medical image.

The respective grid spacing is defined as the distance between two adjacent nodes of the respective grid, wherein the respective node is defined as the intersection of three grid lines and forms a sampling point.

In a further embodiment, the method further comprises:
receiving a query determining the query points for classifying the labels to the sparse sampling descriptors, the query defining the locations and distances of the query points in the volumetric medical image.

In a further embodiment, the method further comprises:
receiving a command, in particular a user input, for adjusting the query, wherein the locations and distances of the query points of the query are adjusted in dependence on the received command.

In a further embodiment, steps d) and e) include:
receiving a first query determining first query points for providing a coarse segmentation mask, the first query defining first locations and first spacings of the first query points in the volumetric medical image,
classifying first labels for the first query points in the volumetric medical image by applying the trained classifier to the obtained sparse sampling descriptors,
providing the coarse segmentation mask for the volumetric medical image using the classified first labels,
receiving a second query determining second query points for providing a fine segmentation mask, the second query defining second locations and second spacings of the second query points in the volumetric medical image, wherein the second spacings are different to the first spacings, in particular smaller than the first spacings,
classifying second labels for the second query points in the volumetric medical image by applying the trained classifier to the obtained sparse sampling descriptors, and
providing the fine segmentation mask for the volumetric medical image using the classified second labels.

For example, the first query may define the first query points such that they have first spacings of 8 mm. By using said first query, the coarse segmentation mask may be provided. Once this mask is generated, there is no additional query needed for the whole image since it covers all locations. However, the coarse segmentation mask may be not accurate in the edges due to low resolution. Since errors may happen in the edges, one could further refine the resolution in those regions by using the second query. To achieve this, all points in the segmentation mask may be checked with higher resolution, and points may be added, if the neighbors having different labels, as different labels define an edge. Thus, the cost of classification in high resolution in the homogeneous points may be eliminated.

Furthermore, a connected component analysis may be included to remove false positive errors in the segmentation masks before refinement. For example, liver appears in the bottom of the lung and some query locations could give liver locations in some small regions on the various locations, for example on top of the lung. In particular, the biggest connected component may be selected to remove erroneous regions before further refining the edges. This may further improve the accuracy and the speed of the present approach. Further, query edge locations by adapting the queries may be repeated until the desired level of granularity is satisfied.

In a further embodiment, the second query is determined such that second query points are selected from neighbors where two of neighbor first query points have different labels. As neighbors having different labels may define an edge, these points in the segmentation mask can be checked with higher resolution.

In a further embodiment, the respective sparse sampling descriptor is formed as a vector of values, in particular of intensities, of the sampled voxels associated to a certain sampling point of the distributed sampling points.

In a further embodiment, the provided segmentation mask or part thereof, in particular comprising the certain point of interest, is displayed on a graphical user interface, wherein the segmentation mask is displayed such that each intensity in the displayed segmentation mask represents a different label, in particular a different organ label.

In a further embodiment, the trained classifier comprises a neural network, in particular a residual neural network. The residual neural network is particularly configured to receive the obtained sparse sampling descriptors and to provide the labels, in particular each of the labels in the form of a vector of estimated probabilities for each organ, as an output.

In particular, the residual neural network comprises a plurality of different layers. The different layers particularly include linear projection, normalization, activation, linear projection and normalization.

In a further embodiment, the obtained N sparse sampling descriptors are decoded into a 2D image including the certain point of interest. In particular, the 2D image is displayed on a graphical user interface together with a plurality of different 3D slices of the volumetric medical image, the different 3D slices having different resolutions and different ranges.

According to a second aspect, a computer-implemented device for automated processing of volumetric medical images is provided, the computer-implemented device comprising:
one or more processing units,
a receiving unit which is configured to receive one or more volumetric medical images captured by a medical imaging unit, and
a memory coupled to the one or more processing units, the memory comprising a module configured to perform the method steps of the first aspect or of any embodiment of the first aspect.

The respective unit, e.g., the processing unit or the receiving unit, may be implemented in hardware and/or in software. If said unit is implemented in hardware, it may be embodied as a device, e.g., as a computer or as a processor or as a part of a system, e.g., a computer system. If said unit is implemented in software, it may be embodied as a computer program product, as a function, as a routine, as a program code or as an executable object.

The embodiments and features according to the first aspect are also embodiments of the second aspect.

According to a third aspect, a system for automated processing of volumetric medical images is provided, the system comprising:
one or more servers,
a medical imaging unit coupled to the one or more servers,
the one or more servers, comprising instructions, which when executed causes the one or more servers to perform the method steps of the first aspect or of any embodiment of the first aspect.

The embodiments and features according to the first aspect are also embodiments of the third aspect.

According to a fourth aspect, a computer program product is provided, the computer program product comprising machine readable instructions, that when executed by one or more processing units, cause the one or more processing units to perform the method steps of the first aspect or of any embodiment of the first aspect.

The embodiments and features according to the first aspect are also embodiments of the fourth aspect.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a fifth aspect, a computer readable medium on which program code sections of a computer program are saved, the program code sections being loadable into and/or executable in a system to make the system execute the method steps of the first aspect or of any embodiment of the first aspect when the program code sections are executed in the system.

The embodiments and features according to the first aspect are also embodiments of the fifth aspect.

The realization by a computer program product and/or a computer-readable medium has the advantage that already existing management systems can be easily adopted by software updates in order to work as proposed by the invention.

"A" is to be understood as non-limiting to a single element. Rather, one or more elements may be provided, if not explicitly stated otherwise. Further, "a", "b" etc. in steps a), step b) etc. is not defining a specific order. Rather, the steps may be interchanged as deemed fit by the skilled person.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a client-server architecture embodying a system for automated processing of volumetric medical images;
- FIG. 2: illustrates a block diagram of a data processing system embodying a device for automated processing of volumetric medical images;
- FIG. 3: illustrates a flowchart of an embodiment of a computer-implemented method for automated processing of volumetric medical images;
- FIG. 4: illustrates a slice from a volumetric medical image and including portions of a sparse sampling model according to an embodiment;
- FIG. 5: shows an example of a volumetric medical image with some voxels being scanned, others being skipped;
- FIG. 6: shows different three-dimensional grids used in the definition of the sparse sampling model of FIG. 4;
- FIG. 7: illustrates a flowchart of an embodiment of a computer-implemented method for identifying a type of organ;
- FIG. 8: illustrates a flowchart of an embodiment of the sampling step and the classifying step of the computer-implemented method of FIG. 3;
- FIG. 9: illustrates an embodiment of a coarse segmentation mask;
- FIG. 10: illustrates the coarse segmentation mask of FIG. 9 additionally including query points of a query;
- FIG. 11: illustrates an embodiment of a fine segmentation mask;
- FIG. 12: illustrates a graphical user interface displaying a 2D image and three different 3D slices of the volumetric medical image; and
- FIG. 13: illustrates a further embodiment of a coarse segmentation mask additionally including query points of a query;

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

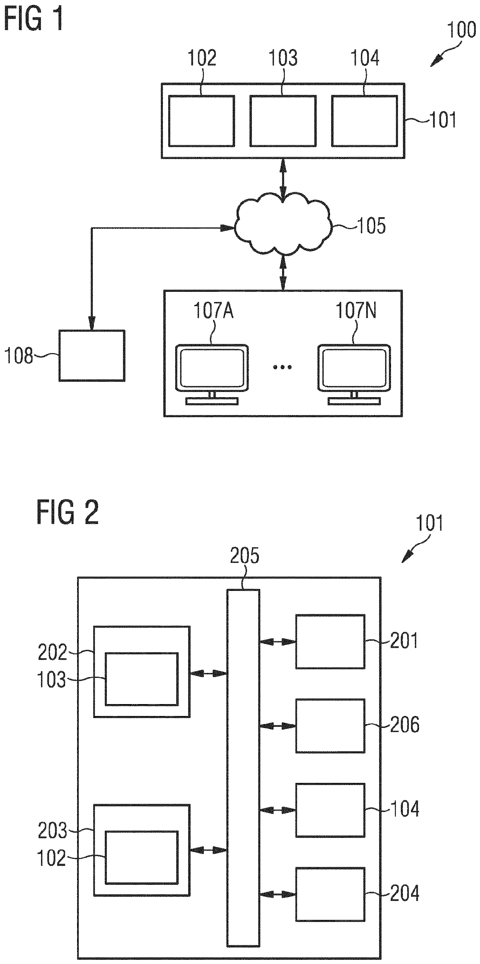

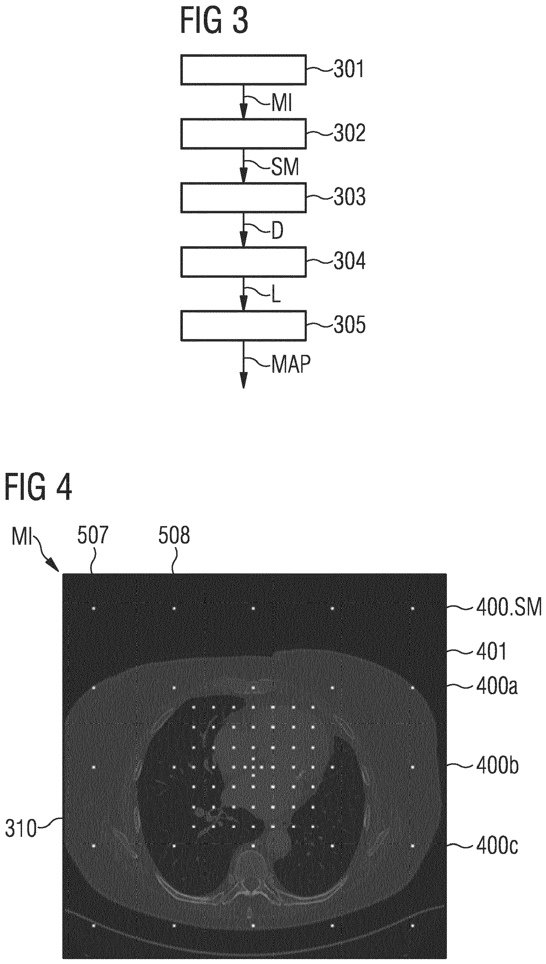

FIG. 1 provides an illustration of a block diagram of a client-server architecture embodying a system for automated processing of volumetric medical images MI (see FIG. 4) The client-server architecture 100 comprises a server 101 and a plurality of client devices 107A-N. Each of the client devices 107A-N is connected to the server 101 via a network 105, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 105, for example, the internet. The cloud computing environment provides on-demand network access to a shared pool of the configurable computing physical and logical resources. The server 101 may include a medical database 102 that comprises medical images related to a plurality of patients that is maintained by a healthcare service provider. In an embodiment, the medical database 102 comprises volumetric medical images captured by a MR scanner and/or by a CT scanner. The server 101 may include a module 103 that is configured to perform identifying a type of organ in a volumetric medical image, in particular as described hereinafter.

The client devices 107A-N are user devices, used by users, for example, medical personnel such as a radiologist, pathologist, physician, etc. In an embodiment, the user device 107A-N may be used by the user to receive volumetric medical images or 2D medical images associated with the patient. The data can be accessed by the user via a graphical user interface of an end user web application on the user device 107A-N. In another embodiment, a request may be sent to the server 101 to access the medical images associated with the patient via the network 105.

An imaging unit 108 may be connected to the server 101 through the network 105. The unit 108 may be a medical imaging unit 108 capable of acquiring a plurality of volumetric medical images. The medical imaging unit 108 may be, for example, a scanner unit such as a magnetic resonance imaging unit, computed tomography imaging unit, an X-ray fluoroscopy imaging unit, an ultrasound imaging unit, etc.

FIG. 2 is a block diagram of a data processing system 101 in which an embodiment can be implemented, for example, as a system 101 for automated processing of volumetric medical images MI, configured to perform the processes as described herein. It is appreciated that the server 101 is an exemplary implementation of the system in FIG. 2. In FIG. 2, said data processing system 101 comprises a processing unit 201, a memory 202, a storage unit 203, an input unit 204, an output unit 206, a bus 205, and a network interface 104.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 comprises a module 103 stored in the form of machine-readable instructions on any of said above-mentioned storage media and may be in communication to and executed by processing unit 201. When executed by the processing unit 201, the module 103 causes the processing unit 201 to provide a segmentation mask and/or to identify a type of organ in a volumetric medical image. Method steps executed by the processing unit 201 to achieve the abovementioned functionality are elaborated upon in detail in the following figures.

The storage unit 203 may be a non-transitory storage medium which stores the medical database 102. The input unit 204 may include input means such as keypad, touch-sensitive display, camera (such as a camera receiving gesture-based inputs), a port etc. capable of providing input signal such as a mouse input signal or a camera input signal. The bus 205 acts as interconnect between the processor 201, the memory 202, the storage unit 203, the input unit 204, the output unit 206 and the network interface 104. The volumetric medical images may be read into the medical database 102 via the network interface 104 or the input unit 204, for example.

Those of ordinary skilled in the art will appreciate that said hardware depicted in FIG. 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 101 in accordance with an embodiment of the present disclosure may comprise an operating system employing a graphical user interface (GUI). Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

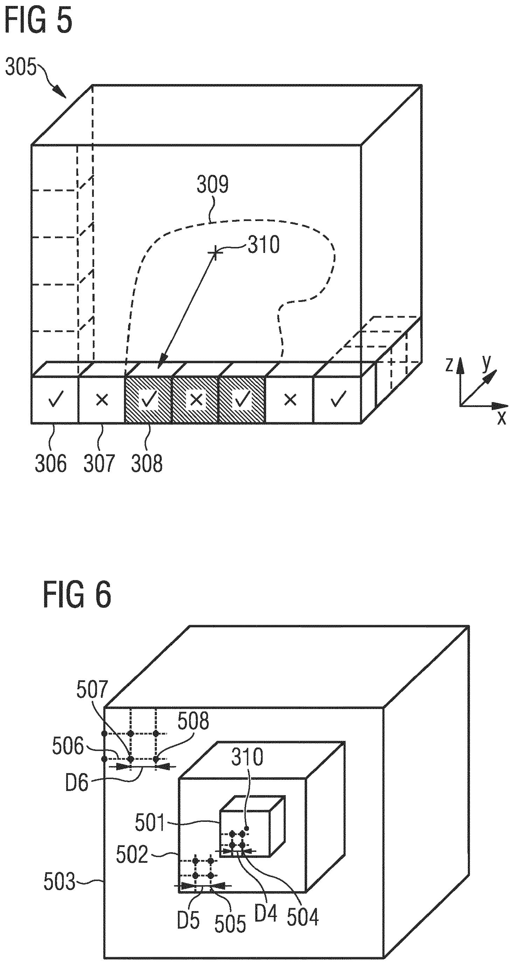

FIG. 3 illustrates a flowchart of an embodiment of a method for automated processing of volumetric medical images MI. The method of FIG. 3 is discussed with reference to FIG. 4, FIG. 5 and FIG. 6. The method of FIG. 3 includes the method steps 301 - 305:
In step 301, a volumetric medical image MI (see FIG. 4 and FIG. 5) is received. In particular, the volumetric medical image MI comprises at least one organ or portion thereof.

The volumetric medical image MI as shown in FIG. 5 is comprised of a three-dimensional array of voxels 306, 307, 308. This array is illustrated in FIG. 5 as a cuboid seen in a perspective view. The cuboid comprises rows and columns of voxels 306, 307, 308 extending in all three dimensions x, y, z. To make FIG. 5 more readable, only some of the voxels 306, 307, 308 within the cuboid are shown.

Instead of the three-dimensional array, the method explained herein may also use a number of slices (two-dimensional arrays of pixels) which, taken together, describe a (three-dimensional) volume. In fact, any other data structure may be used comprising values, such as intensities, and describing a three-dimensional space. Any such value is termed a "voxel" herein. The value may be combined with information describing its three-dimensional relationship with respect to other values, or the three-dimensional relationship can be inferred from the data structure, or any other source.

The volumetric medical image MI comprises at least one organ 309 or a portion thereof. In the example of FIG. 5, a portion of an organ 309, for example lungs or kidney, is represented by hashed voxels 308 in the volumetric medical image MI.

In FIG. 5, a point of interest 310 within the volumetric medical image MI is illustrated. As will be explained in more detail later, the point of interest 310 may be selected by a user, for example, through a graphical user interface using a pointer device, such as a mouse. The point of interest 310 may be received, for example, through the network interface 104 or the input unit 204 (see FIG. 2).

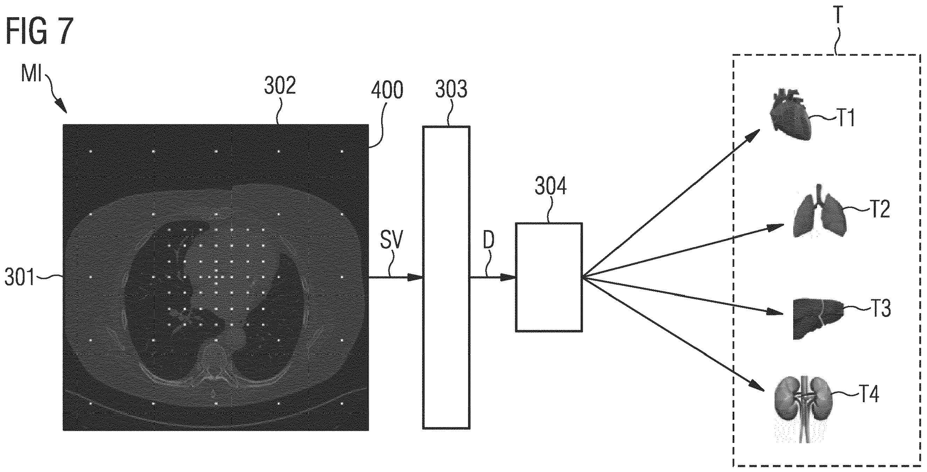

The point of interest 310 is a point in the volumetric medical image MI for which it may be desired to identify the type of organ 309 corresponding to said point (see FIG. 7). Said single point of interest 310 may be described through coordinates in x, y, z and may correspond to a specific voxel in the cuboid. If, for example, a mouse is used to select the point of interest 310, the coordinates of the mouse cursor, once the selection of the single point of interest 310 is done, for example by clicking or pausing the cursor over the image, said coordinates x, y, z are transmitted, for example, via the input unit 204 to the data processing system 101.

In step 302, a sparse sampling model SM for sparse sampling the volumetric medical image MI is provided. As shown in FIG. 4, the sparse sampling model SM defines a number N of sampling points 400a-400c distributed in the volumetric medical image MI and defining locations and distances of the distributed sampling points 400a-400c.

In particular, the sparse sampling model SM is provided such that the voxels 306-308 are sampled with a sampling rate per unit length, area or volume which decreases with a distance 311 (see FIG. 5) of the respective voxel 306-308 on the point of interest 310. According to the sparse sampling model SM, at least one voxel 307 is skipped between two voxels 306, 308 that are to be sampled. In FIG. 5, the voxels that are to be sampled are designated with a tick, whereas voxels that are skipped or not sampled are designated with a cross. In the embodiment of FIG. 5, in the row of voxels defined by adjacent voxels 306, 307, 308 only every second voxel 306, 308 is sampled. The voxels 306, 308 may be sampled sequentially or in parallel.

In particular, the inventors found that it is beneficial if the voxels 306, 308 that are to be sampled are sampled (in the following step 303) with a sampling rate per unit length, area or volume which decreases with a distance 311 from the point of interest 310. It was found that results improve even more, when the sampling rate decreases at a nonlinear rate, in particular at the rate of an exponential, logarithmic or power function.

In this regard, it is referred to FIGS. 4 and 6 which show an example of a sparse sampling model SM (only some of the sampling points of the sampling model SM have been marked with reference numerals 400a, 400b, 400c for reasons of readability of FIG. 4) in a 2D slice 401 taken from a volumetric medical image MI. The sampling model 400 is designed to provide for a sparse sampling with a sampling rate per unit volume which decreases with a distance in the x-, y- and z-direction. Since FIG. 4 shows a 2D image, the y-direction which corresponds to the depth normal to the plane of the image shown in FIG. 4 is not illustrated but corresponds to the sampling rate shown for the directions x and z in FIG. 4.

FIG. 6 illustrates three 3D grids 504, 505, 506 of different grid spacings used in the definition of the sparse sampling model SM of FIG. 4. Thus, as shown in FIG. 6, the sparse sampling model SM defines a plurality of grids 504, 505, 506, in particular 3D grids, of different grid spacings, the different grid spacings determining different distances D4, D5, D6 of the distributed sampling points 400a-400c in the volumetric medical image MI. With reference to FIG. 6, the cubes (or cuboids) are designated 501, 502 and 503. The cubes 501, 502, 503 are nested within each other, with the smallest cube 501 containing, for example at its center, the point of interest 310. Each cube contains a grid, respectively. The cube 501 contains a grid 504, the cube 502 a grid 505 and the cube 503 a grid 506. The grids 504, 505, 506 may have a regular spacing defining cubes or cuboids within. For illustration purposes, the grids 504, 505, 506 are only shown in 2D (i.e., in the plane of the paper) and only partially. The grid spacing is defined as the distance between two adjacent nodes of the respective grid. Any node is defined as the intersection of three grid lines. As shown in more detail for grid 506, two nodes 507, 508 are a distance D6 apart. Similarly, the grid spacing of the grid 504 is defined by a distance D4, and the grid spacing of the grid 505 is defined by a distance D5.

In the experiment made by the inventors, D4 was selected 8 mm, D5 20 mm and D6 80 mm. The nodes 507, 508 considered were only those nodes within the volume of each cube (or cuboids) minus the volume of the largest cube (or cuboid) nested in-side said cube. For example, for cube 503, the nodes 507, 508 were considered in-side the volume of the cube 503 which were not lying in the volume of the cube 502.

The nodes 507, 508 define the sampling model SM and thus define the voxels 306, 308 (see FIG. 5) that are to be sampled in the volumetric medical image MI. Thus, when sampling (in the following step 303) the volumetric medical image MI with the sampling model SM, 1,195 voxels are sampled (that is, for example, their intensity values read from the database 102), whereas the total number of voxels in the volumetric medical image was 25,000,000. Thus, less than 0.1% of the total number of voxels are sampled.

In step 303, voxels 306, 308 (see FIG. 5) from the volumetric medical image MI are sampled using the provided sparse sampling model SM for obtaining N sparse sampling descriptors D. The respective sparse sampling descriptor D may be formed as a vector of values, in particular of intensities, of the sampled voxels associated to a certain sampling point 400a-400c of the distributed sampling points 400a-400c.

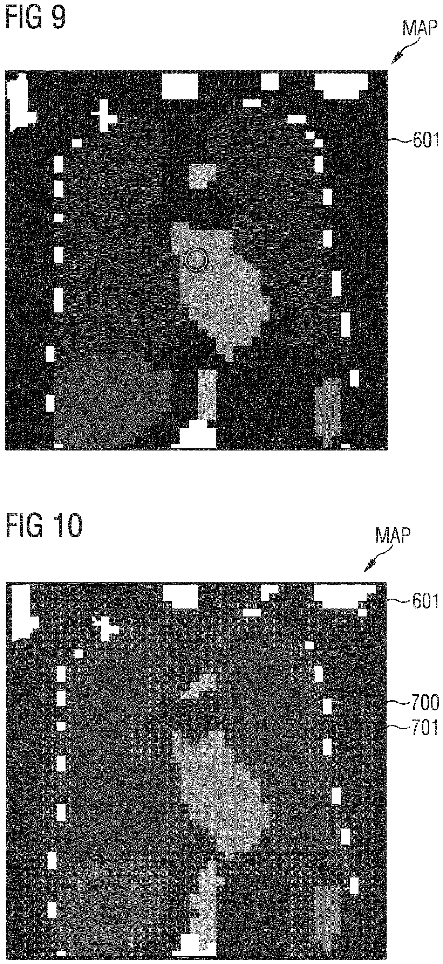

In step 304, labels L for query points 701 (see FIG. 10) in the volumetric medical image MI are classified by applying a trained classifier to the obtained sparse sampling descriptors D. In this regard, a query 700 may be received, said query 700 determining the query points 701 for classifying the labels L to the sparse sampling descriptors D. In particular, the query 700 defines the locations and distances of the query points 701 in the volumetric medical image MI. In embodiments, the method may further comprise a step of receiving a command, in particular a user input, for adjusting the query 700. Here, the locations and distances of the query points 701 of the query 700 may be adjusted in dependence on the received command.

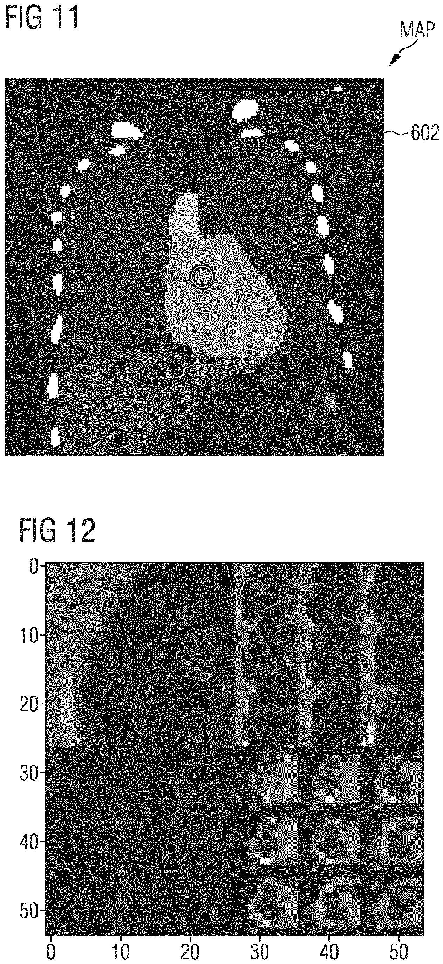

In step 305, a segmentation mask MAP, 601, 602 is provided for the volumetric medical image MI using the classified labels L. In FIG. 3, reference numeral MAP designates the segmentation mask. Furthermore, FIG. 9 shows a coarse segmentation mask 601, and FIG. 11 shows a fine segmentation mask 602. The segmentation mask MAP, 601, 602 may be used for identifying organ boundaries, displaying visualizations or volume calculations.

Further, FIG. 7 shows a flowchart of an embodiment of a computer-implemented method for identifying a type of organ T1- T4. The method of FIG. 7 comprises method steps 301 - 304 corresponding to method steps 301 - 304 as discussed with reference to FIG. 3. Further, the method of FIG. 7 may optionally comprise method step 305 as discussed with reference to FIG. 3, i.e., the step of providing a segmentation mask 601, 602 for the volumetric medical image MI using the classified labels L (not shown in FIG. 7).

Moreover, the method of FIG. 7 comprises a step 305b of identifying the type of organ T1 - T4 at a certain point of interest 310, in particular by applying the trained classifier to the sampled voxels SV and/or to the obtained sparse sampling descriptors D. Furthermore, the method of FIG. 7 may include the step of receiving a command for determining the certain point of interest 310. Then, the sparse sampling model SM may be provided in dependence on the received command or may be adapted in dependence on the received command. The command may be a certain user input.

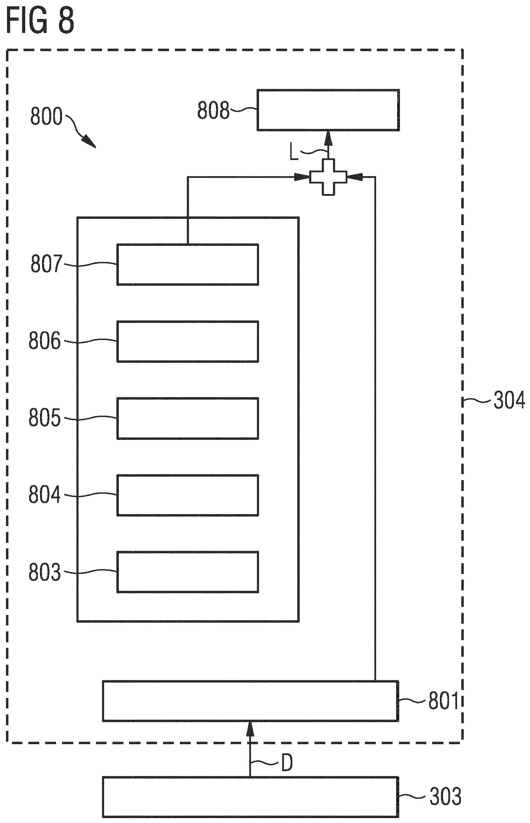

Moreover, FIG. 8 is a flowchart of an embodiment of the sampling step 303 and the classifying step 304 of the computer-implemented method of FIG. 3. As discussed with reference to FIG. 3, the sampling step 303 outputs N sparse sampling descriptors D. Further, as shown in FIG. 3 as well as in FIG. 8, the classifying step 304 receives the N sparse sampling descriptors D. The classifying step 304 classifies labels L for query points 701 in the volumetric medical image MI by applying a trained classifier to the N sparse sampling descriptors D. The trained classifier may comprise a neural network 800, in particular a residual neural network 800 as depicted in FIG. 8. The residual neural network 800 as shown in FIG. 8 is particularly configured to receive the obtained sparse sampling descriptors D and to provide the labels L. In particular, each of the labels L is in the form of a vector of estimated probabilities for each organ.

As shown in FIG. 8, the residual neural network 800 may comprise a plurality of different layers, particularly including dimension reduction 801, linear projection 802, normalization 803, activation 804, linear projection 805 and normalization 806. The labels L in the form of the vector of estimated probabilities for each organ may be input into an identifying layer 807 which is adapted to identify the type of organ T1 - T4 based on the received labels L.

Moreover, an embodiment of the present method of FIG. 3 for automated processing of volumetric medical images MI is discussed in the following with reference to FIGS. 9 - 11. In this regard, FIG. 9 illustrates an embodiment of a coarse segmentation mask 601 as a potential output of the method of FIG. 3, FIG. 10 shows the coarse segmentation mask 601 of FIG. 9 additionally including query points 701 of a query 700, and FIG. 11 shows an embodiment of a fine segmentation mask 602 as a potential output of the method of FIG. 3. As discussed above, a query 700 may determine query points 701 for classifying the labels L to the sparse sampling descriptors D, wherein the query 700 defines locations and distances of the query points 701 in the volumetric medical image MI (see FIG. 10). To make FIG. 10 more readable, only one query point 701 is referenced by a reference numeral. All the other query points in FIG. 10 (small white points) become apparent when comparing FIG. 10 with FIG. 9.

The mechanism how a user can output a coarse segmentation mask 601 as shown in FIG. 9 or a fine segmentation mask 602 as shown in FIG. 11 is explained in the following: This mechanism is based on embodiments of the above discussed classifying step 304 and the providing step 305 of FIG. 3, said embodiments including the following steps:
receiving a first query determining first query points for providing a coarse segmentation mask 601 (see FIG. 9), the first query defining first locations and first spacings of the first query points in the volumetric medical image MI,
classifying first labels for the first query points in the volumetric medical image MI by applying the trained classifier to the obtained sparse sampling descriptors D,
providing the coarse segmentation mask 601 (see FIG. 9) for the volumetric medical image MI using the classified first labels,
receiving a second query 700 (see FIG. 10) determining second query points 701 for providing a fine segmentation mask 602, the second query 700 defining second locations and second spacings of the second query points 701 in the volumetric medical image MI, wherein the second spacings are different to the first spacings, in particular smaller than the first spacings,
classifying second labels for the second query points 701 in the volumetric medical image MI by applying the trained classifier to the obtained sparse sampling descriptors D, and
providing the fine segmentation mask 602 (see FIG. 11) for the volumetric medical image MI using the classified second labels.

Thus, by defining the query points 701 as apparent by comparing FIGS. 9 and 11, the user may define the segmentation mask to be output, for example a coarse segmentation mask 601 as shown in FIG. 9 or a fine segmentation mask 602 as shown in FIG. 11. As shown in FIGS. 9 - 11, the respective segmentation mask 601, 602 or part thereof may be displayed on a graphical user interface (GUI). The segmentation mask 601, 602 may be displayed such that each intensity in the displayed segmentation mask 601, 602 represents a different label, in particular a different organ label.

Moreover, the N sparse sampling descriptors D as obtained by above-discussed step 303 may be decoded into a 2D image 1201 including the point of interest 310, wherein the 2D image 1201 may be displayed on a graphical user interface - as shown in FIG. 12 - together with a plurality of different 3D slices 1202 - 1204 of the volumetric medical image MI. Without loss of generality, the plurality of different 3D slices 1202 - 1204 is three in FIG. 12. The different 3D slices 1202 - 1204 may have different resolutions and different ranges.

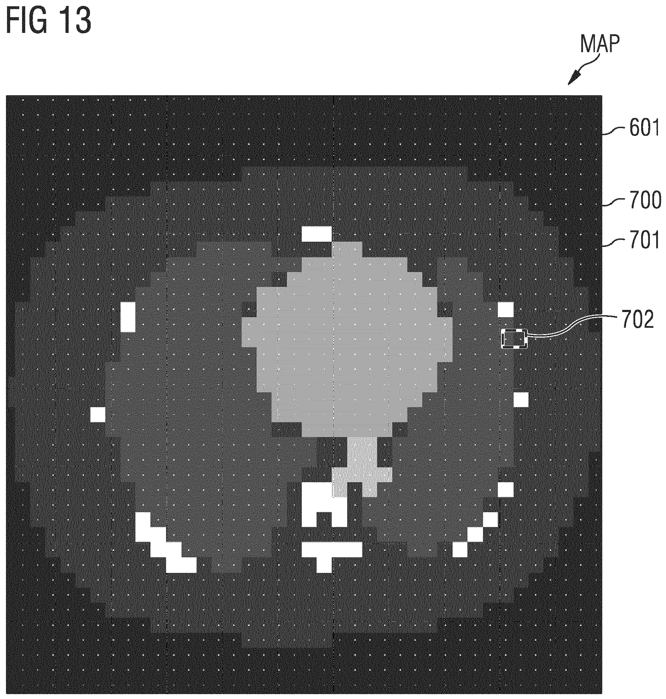

Moreover, FIG. 13 illustrates a further embodiment of a coarse segmentation mask 601 additionally including query points 701 of a query 700. In the embodiment of FIG. 13, the query 700 is determined such that query points 701 are selected from neighbors where two of neighbor first query points have different labels. To make FIG. 13 more readable, only one pair 702 of neighbors having different labels is referenced by a white, dashed box having the reference numeral 702. As neighbors 702 having different labels may define an edge, these points in the segmentation mask can be checked with higher resolution.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### REFERENCES

[1] https://flare.grand-challenge.org/
[2] Zhang, Fan, Yu Wang, and Hua Yang. "Efficient Context-Aware Network for Abdominal Multi-organ Segmentation." arXiv preprint arXiv:2109.10601 (2021).
[3] Xie, Enze, et al. "SegFormer: Simple and efficient design for semantic segmentation with transformers." Advances in Neural Information Processing Systems 34 (2021): 12077-12090.
[4] Ronneberger, O., Fischer, P., & Brox, T. (2015, October). U-net: Convolutional networks for biomedical image segmentation. In International Conference on Medical image computing and computer-assisted intervention (pp. 234-241). Springer, Cham.
[5] Gibson, Eli, et al. "Automatic multi-organ segmentation on abdominal CT with dense v-networks." IEEE transactions on medical imaging 37.8 (2018): 1822-1834.
[6] Chen, J., Lu, Y., Yu, Q., Luo, X., Adeli, E., Wang, Y., ... & Zhou, Y. (2021). Transunet: Transformers make strong encoders for medical image segmentation. arXiv preprint arXiv:2102.04306.
[7] Tang, Y., Yang, D., Li, W., Roth, H. R., Landman, B., Xu, D., ... & Hata-mizadeh, A. (2022). Self-supervised pre-training of swin transformers for 3d medical image analysis. In Proceedings of the IEEE/CVF Conference on Computer Vision and Pattern Recognition (pp. 20730-20740).
[8] EP 22 182 378.4

### REFERENCE SIGNS

- 100: system
- 101: computer-implemented device
- 102: medical database
- 103: module
- 104: network interface
- 105: network
- 107A - 107N: client device
- 108: medical imaging unit
- 201: processing unit
- 202: memory
- 203: storage unit
- 204: input unit
- 205: bus
- 206: output unit
- 301 - 305: method steps
- 305b: method step
- 306 - 308: voxels
- 309: organ
- 310: single point of interest
- 311: distance
- 400: sampling model
- 400a - 400c: sampling points
- 401: slice
- 501 - 503: cubes
- 504 - 506: grids
- 507, 508: nodes
- 601: coarse segmentation mask
- 602: fine segmentation mask
- 700: query
- 701: query point
- 702: pair of neighbors having different labels
- 800: neural network
- 801 - 807: layer
- 1201: 2D image
- 1202: 3D slice
- 1203: 3D slice
- 1204: 3D slice

- D: sparse sampling descriptor
- D4, D5, D6: distances
- GUI: graphical user interface
- L: label
- MAP: segmentation mask
- MI: medical image
- SM: sampling model
- SV: sampled voxels
- T1: first type of organ
- T2: second type of organ
- T3: third type of organ
- T4: fourth type of organ
- x, y, z: orthogonal directions in space

## Claims

**1.** A computer-implemented method for automated processing of volumetric medical images (MI), the method comprising:
a) receiving (301) a volumetric medical image (MI), the volumetric medical image (MI) comprising at least one organ or portion thereof,
b) providing (302) a sparse sampling model (SM) for sparse sampling the volumetric medical image (MI), the sparse sampling model (SM) defining a number N of sampling points (400a-400c) distributed in the volumetric medical image (MI) and defining locations and distances of the distributed sampling points (400a-400c),
c) sampling (303) voxels (306-308) from the volumetric medical image (MI) using the provided sparse sampling model (SM) for obtaining N sparse sampling descriptors (D),
d) classifying (304) labels (L) for query points (701) in the volumetric medical image (MI) by applying a trained classifier to the obtained sparse sampling descriptors (D), and
e) providing (305) a segmentation mask (MAP, 601, 602) for the volumetric medical image (MI) using the classified labels (L).

**2.** The method of claim 1, further comprising:
identifying the type of organ (T, T1-T4) at a certain point of interest (310), in particular by applying the trained classifier to the sampled voxels (SV).

**3.** The method of claim 2, further comprising:
receiving a command, in particular a user input, for determining the certain point of interest (310), wherein the sparse sampling model (SM) is provided in dependence on the received command.

**4.** The method of claim 3,
wherein the sparse sampling model (SM) is provided such that the voxels (306-308) are sampled with a sampling rate per unit length, area or volume which decreases with a distance (311) of the respective voxel (306-308) on the certain point of interest (310).

**5.** The method of one of the claims 1 to 4,
wherein the sparse sampling model (SM) defines a plurality of grids (504, 505, 506), in particular 3D grids, of different grid spacings, the different grid spacings determining different distances (D4, D5, D6) of the distributed sampling points (400a-400c) in the volumetric medical image (MI).

**6.** The method of one of the claims 1 to 5, further comprising:
receiving a query (700) determining the query points (701) for classifying the labels (L) to the sparse sampling descriptors (D), the query (700) defining the locations and distances of the query points (701) in the volumetric medical image (MI).

**7.** The method of claim 6, further comprising:
receiving a command, in particular a user input, for adjusting the query (700), wherein the locations and distances of the query points (701) of the query (700) are adjusted in dependence on the received command.

**8.** The method of one of claims 1 to 7, wherein steps d) and e) include:
receiving a first query determining first query points for providing a coarse segmentation mask (601), the first query defining first locations and first spacings of the first query points in the volumetric medical image (MI),
classifying first labels for the first query points in the volumetric medical image (MI) by applying the trained classifier to the obtained sparse sampling descriptors (D),
providing the coarse segmentation mask (601) for the volumetric medical image (MI) using the classified first labels,
receiving a second query (700) determining second query points (701) for providing a fine segmentation mask (602), the second query (700) defining second locations and second spacings of the second query points (701) in the volumetric medical image (MI), wherein the second spacings are different to the first spacings, in particular smaller than the first spacings,
classifying second labels for the second query points (701) in the volumetric medical image (MI) by applying the trained classifier to the obtained sparse sampling descriptors (D), and
providing the fine segmentation mask (602) for the volumetric medical image (MI) using the classified second labels.

**9.** The method of one of claims 1 to 8,
the second query (700) is determined such that second query points (701) are selected from neighbors where two of neighbor first query points have different labels.

**10.** The method of one of claims 1 to 9,
wherein the respective sparse sampling descriptor (D) is formed as a vector of values, in particular of intensities, of the sampled voxels (SV) associated to a certain sampling point (400a-400c) of the distributed sampling points (400a-400c).

**11.** The method of one of claims 1 to 10,
wherein the provided segmentation mask (MAP, 601, 602) or part thereof, in particular comprising the certain point of interest (310), is displayed on a graphical user interface, wherein the segmentation mask (601, 602) is displayed such that each intensity in the displayed segmentation mask (601, 602) represents a different label, in particular a different organ label.

**12.** The method of one of claims 1 to 11,
wherein the trained classifier comprises a neural network (800), in particular a residual neural network (800), wherein the residual neural network (800) is particularly configured to receive the obtained sparse sampling descriptors (D) and to provide the labels (L), in particular each of the labels in the form of a vector of estimated probabilities for each organ, as an output.

**13.** The method of one of claims 1 to 12,
wherein the obtained N sparse sampling descriptors (D) are decoded into a 2D image including the certain point of interest (310), wherein the 2D image is displayed on a graphical user interface together with a plurality of different 3D slices of the volumetric medical image (MI), the different 3D slices having different resolutions and different ranges.

**14.** A computer-implemented device (101) for automated processing of volumetric medical images (MI), the computer-implemented device (101) comprising:
one or more processing units (201),
a receiving unit (204) which is configured to receive one or more volumetric medical images (MI) captured by a medical imaging unit (108), and
a memory (202) coupled to the one or more processing units (201), the memory (202) comprising a module (103) configured to perform the method steps as claimed in any one of claims 1 to 13.

**15.** A system (100) for automated processing of volumetric medical images (MI), the system (100) comprising:
one or more servers (101),
a medical imaging unit (108) coupled to the one or more servers (101), the one or more servers (101) comprising instructions, which when executed causes the one or more servers (101) to perform the method steps as claimed in any one of claims 1 to 13.

**15.** A computer program product comprising machine readable instructions, that when executed by one or more processing units (201), cause the one or more processing units (201) to perform method steps according to claims 1 to 13.

**16.** A computer readable medium on which program code sections of a computer program are saved, the program code sections being loadable into and/or executable in a system (100) to make the system (100) execute the method steps according to any one of the claims 1 to 13 when the program code sections are executed in the system (100).
